# FASCICULE DE BREVET EUROPEEN

(11) **EP 2 598 240 B1**
(45) Date de publication et mention de la délivrance du brevet: **12.08.2015**
(21) Numéro de dépôt: 11743263.3
(22) Date de dépôt: 24.06.2011
(51) Int. Cl.: B01J 23/75, B01J 37/02, C07C 1/04, C10G 2/00, B01J 31/06, B01J 21/00, B01J 21/04, B01J 21/12, B01J 23/00, B01J 37/12, B01J 23/89, B01J 35/00, B01J 35/02, B01J 35/10

(54) **PROCEDE DE SYNTHESE D'HYDROCARBURES EN C5+ EN PRESENCE D'UN CATALYSEUR PREPARE AU MOYEN D'AU MOINS UN OLIGOSACCHARIDE CYCLIQUE**
VERFAHREN ZUR SYNTHESE VON C5+KOHLENWASSERSTOFFEN IN GEGENWART EINES MIT MINDESTENS EINEM CYCLISCHEN OLIGOSACCHARID HERGESTELLTEN KATALYSATORS
METHOD FOR SYNTHESIZING C5+ HYDROCARBONS IN THE PRESENCE OF A CATALYST PREPARED USING AT LEAST ONE CYCLIC OLIGOSACCHARIDE

(30) Priorité: 29.07.2010 FR 1003186
(43) Date de publication de la demande: 05.06.2013
(73) Titulaire: IFP Energies nouvelles, 92852 Rueil-Malmaison Cedex (FR)
(72) Inventeur: DIEHL, Fabrice, F-69007 Lyon (FR); GRIBOVAL-CONSTANT, Anne, F-59650Villeneuve d'Ascq (FR); KHODAKOV, Andrei, F-59170 Croix (FR); JAEN-MARIE, Alan, F-62231 Sangatte (FR); MONFLIER, Eric, F-59110 La Madeleine (FR)
(86) Numéro de dépôt international: PCT/FR2011/000369
(87) Numéro de publication internationale: WO 2012/013866

(56) Documents cités:
- US-A- 5 856 261
- US-A1- 2005 040 090

## Description

### Domaine de l'invention

La présente invention se rapporte au domaine des procédés de synthèse Fischer-Tropsch qui permettent d'obtenir une large gamme de coupes hydrocarbonées à partir du mélange CO + H₂, communément appelé gaz de synthèse ou syngaz.

L'équation stoechiométrique simplifiée (limitée dans l'équation ci-dessous à la formation d'alcanes) de la synthèse Fischer-Tropsch s'écrit :

n CO + (2n+1) H₂ -> CₙH₂ₙ₊₂ + n H₂O

Cette réaction est généralement réalisée à moyenne ou haute température et sous pression.

### Examen de l'art antérieur

Les catalyseurs utilisés en synthèse Fischer-Tropsch sont le plus souvent des catalyseurs supportés à base d'alumine, d'alumine silicée, de silice-alumine, de silice, de dioxyde de titane, de zircone ou de combinaisons de ces supports, la phase active étant principalement constituée de fer (Fe) ou de cobalt (Co) éventuellement dopée par un métal noble tel que le Pt, le Rh ou le Ru.

Il est bien connu de l'Homme du métier que la préparation de catalyseurs pour la synthèse Fischer-Tropsch par les méthodes classiques, par exemple par imprégnation à sec, conduisent à des catalyseurs dont la taille moyenne en oxyde de métal, particulièrement en oxyde de cobalt (Co₃O₄), est souvent élevée et mal contrôlée (grande disparité en tailles de cristallites d'oxyde de métal, particulièrement en cristallites d'oxyde de cobalt Co₃O₄). Il en résulte des catalyseurs présentant une activité catalytique médiocre en synthèse Fischer-Trospch, les performances catalytiques pouvant varier selon l'état d'agrégation du métal, particulièrement du cobalt.

De nombreuses études ont été menées en vue d'augmenter la dispersion du métal, notamment le cobalt, présent dans la phase active des catalyseurs de synthèse Fischer-Tropsch de manière à obtenir des catalyseurs plus actifs. Dans ce but, il a fréquemment été enseigné d'introduire un composé organique lors de la préparation du catalyseur. En particulier, les brevets US 5.856.260 et US 5.856.261 enseignent respectivement l'introduction, lors de la préparation du catalyseur, de polyols de formule générale CₙH₂ₙ₊₂Oₓ avec n un nombre entier compris entre 2 et environ 6, et x un entier compris entre 2 et 11 ou de sucres de type mono- ou disaccharides, le sucrose étant particulièrement préféré. La demande de brevet US 2005/0026776 enseigne l'utilisation de composés chélatants de type acide nitrilotriacétique (NTA), acide trans-1,2-cyclohexadiamine-N,N,N',N' tétraacétique (CyDTA) ou acide éthylènediaminetétraacétique (EDTA), ou encore de glycine, d'acide aspartique ou acide citrique pour l'obtention d'un catalyseur à taille réduite de cristallites Co₃O₄. La demande de brevet US 2002/0082166 enseigne l'utilisation d'une solution contenant un dérivé organoammonium de formule R₁R₂R₃R₄N-OH (où R₁ à R₄ sont des chaînes hydrocarbonées ou des atomes d'hydrogène) ou encore de l'hydroxyde d'ammonium NH₄OH pour obtenir un catalyseur Fischer-Tropsch à base de cobalt plus actif et plus sélectif en produits lourds.

Malgré des améliorations apportées aux catalyseurs utilisés en synthèse Fischer-Tropsch, ceux-ci ne permettent que rarement de conduire à des performances catalytiques optimisées, notamment en terme d'activité catalytique. Aussi, la présente invention se propose de fournir un nouveau procédé de synthèse d'hydrocarbures conduisant à des performances améliorées par rapport à celles obtenues avec des catalyseurs préparés selon les méthodes antérieures.

### Résumé et intérêt de l'invention

La présente invention a pour objet un procédé de synthèse d'hydrocarbures essentiellement linéaires et saturés C5+ consistant en la mise en contact d'une charge comprenant du gaz de synthèse avec au moins un catalyseur dont la phase active comprend au moins un métal du groupe VIII déposé sur un support formé d'au moins un oxyde, ledit catalyseur étant préparé selon un procédé comprenant au moins :
i) au moins une étape de mise en contact d'au moins dudit support avec au moins une solution contenant au moins un précurseur dudit métal du groupe VIII,
ii) au moins une étape de mise en contact d'au moins dudit support avec au moins un composé organique formé d'au moins un oligosaccharide cyclique composé d'au moins 6 sous-unités glucopyranose liées en α-(1,4),
iii) au moins une étape de calcination pour obtenir au moins ledit métal dudit groupe VIII sous forme oxyde,
les étapes i) et ii) pouvant être réalisées séparément, dans un ordre indifférent, ou simultanément.

Conformément au procédé de synthèse selon l'invention, ledit métal du groupe VIII présent dans la phase active du catalyseur est préférentiellement le cobalt. Il est avantageux d'utiliser un catalyseur dont la phase active est formée de deux métaux, de préférence le cobalt et le platine.

Conformément au procédé de synthèse selon l'invention, ledit catalyseur est préférentiellement préparé en présence d'une cyclodextrine en tant que composé organique. De façon surprenante, il a été découvert qu'un catalyseur, dont la phase active comprend au moins un métal du groupe VIII, particulièrement le cobalt, et préparé en présence d'au moins un composé organique formé d'au moins un oligosaccharide cyclique composé d'au moins 6 sous-unités glucopyranose liées en α-(1,4), préférentiellement d'une cyclodextrine, présente une dispersion dudit métal du groupe VIII sensiblement supérieure à celle présentée par des catalyseurs préparés en l'absence d'oligosaccharide cyclique en raison d'une diminution de la taille (diamètre) des cristallites d'oxyde de métal du groupe VIII présent dans la phase active du catalyseur. Il en résulte la présence d'un plus grand nombre de sites actifs pour les catalyseurs préparés en présence d'au moins un oligosaccharide cyclique composé d'au moins 6 sous-unités glucopyranose liées en α-(1,4). Mis en oeuvre dans un procédé de synthèse Fischer-Tropsch, de tels catalyseurs, préparés en présence d'au moins un oligosaccharide cyclique composé d'au moins 6 sous-unités glucopyranose liées en α-(1,4), conduisent à des performances catalytiques améliorées, en termes d'activité catalytique et de productivité envers les produits hydrocarbonés désirés en C5+.

### Description de l'invention

La présente invention a pour objet un procédé de synthèse d'hydrocarbures essentiellement linéaires et saturés C5+ consistant en la mise en contact d'une charge comprenant du gaz de synthèse avec au moins un catalyseur dont la phase active comprend au moins un métal du groupe VIII déposé sur un support formé d'au moins un oxyde, ledit catalyseur étant préparé selon un procédé comprenant au moins :
i) au moins une étape de mise en contact d'au moins dudit support avec au moins une solution contenant au moins un précurseur dudit métal du groupe VIII,
ii) au moins une étape de mise en contact d'au moins dudit support avec au moins un composé organique formé d'au moins un oligosaccharide cyclique composé d'au moins 6 sous-unités glucopyranose liées en α-(1,4),
iii) au moins une étape de calcination pour obtenir au moins ledit métal dudit groupe VIII sous forme oxyde,
les étapes i) et ii) pouvant être réalisées séparément, dans un ordre indifférent, ou simultanément.

Le procédé selon l'invention conduit à la production d'hydrocarbures essentiellement linéaires et saturés C5+. Conformément à l'invention, on entend par hydrocarbures essentiellement linéaires et saturés C5+, des hydrocarbures dont la proportion en composés hydrocarbonés ayant au moins 5 atomes de carbone par molécule représente au moins 50% en poids, de préférence au moins 80% en poids de l'ensemble des hydrocarbures formés, la teneur en composés oléfiniques présents parmi lesdits composés hydrocarbonés ayant au moins 5 atomes de carbone par molécule étant inférieure à 10% poids. Les hydrocarbures produits par le procédé de l'invention sont ainsi des hydrocarbures essentiellement paraffiniques, dont la fraction présentant les points d'ébullition les plus élevés peut être convertie avec un rendement élevé en distillats moyens (coupes gasoil et kérosène) par un procédé d'hydroconversion tel que l'hydrocraquage et/ou l'hydroisomérisation catalytique(s).

La charge employée pour la mise en oeuvre du procédé de l'invention comprend, de préférence est constituée, du gaz de synthèse. Le gaz de synthèse est un mélange de monoxyde de carbone et d'hydrogène présentant des rapports molaires H₂/CO pouvant varier dans un rapport de 0,5 à 4 en fonction du procédé par lequel il a été obtenu. Le rapport molaire H₂/CO du gaz de synthèse est généralement voisin de 3 lorsque le gaz de synthèse est obtenu à partir du procédé de vaporeformage d'hydrocarbures ou d'alcool. Le rapport molaire H₂/CO du gaz de synthèse est de l'ordre de 1,5 à 2 lorsque le gaz de synthèse est obtenu à partir d'un procédé d'oxydation partielle. Le rapport molaire H₂/CO du gaz de synthèse est généralement voisin de 2,5 lorsqu'il est obtenu à partir d'un procédé de reformage thermique. Le rapport molaire H₂/CO du gaz de synthèse est généralement voisin de 1 lorsqu'il est obtenu à partir d'un procédé de gazéification et de reformage du CO₂.

Le catalyseur utilisé dans le procédé de synthèse d'hydrocarbures selon l'invention peut être mis en oeuvre dans différents types de réacteurs, par exemple en lit fixe, en lit mobile, en lit bouillonnant ou encore en lit fluidisé triphasique. La mise en oeuvre du catalyseur en suspension dans un réacteur fluidisé triphasique, préférentiellement de type colonne à bulle, est préférée. Dans cette mise en oeuvre préférée du catalyseur, ledit catalyseur est divisé à l'état de poudre très fine, particulièrement de l'ordre de quelques dizaines de microns, cette poudre formant une suspension avec le milieu réactionnel. Cette technologie est également connue sous la terminologie de procédé "slurry" par l'homme du métier.

Le catalyseur employé pour la mise en oeuvre du procédé de synthèse d'hydrocarbures selon l'invention comprend une phase métallique active déposée sur un support, ladite phase active comprenant au moins un métal du groupe VIII préférentiellement choisi parmi le cobalt, le nickel, le ruthénium et le fer. Dans le cas où la phase active comprend au moins un métal choisi parmi le cobalt, le nickel et le fer, la teneur en ledit métal représente de 1 à 60 % poids par rapport au poids du catalyseur, de préférence de 5 à 30 % poids par rapport au poids du catalyseur et de manière très préférée de 10 à 30% poids par rapport au poids du catalyseur. Dans le cas où la phase active comprend du ruthénium, la teneur en ruthénium est comprise entre 0,01 et 10% poids par rapport au poids du catalyseur et de manière très préférée entre 0,05 et 5% poids par rapport au poids du catalyseur. Ladite phase active déposée sur ledit support comprend avantageusement un métal choisi parmi le cobalt, le nickel, le ruthénium et le fer ou plusieurs métaux choisis parmi le cobalt, le nickel, le ruthénium et le fer. De manière très préférée, ladite phase active comprend du cobalt. La phase active dudit catalyseur comprend également de manière avantageuse au moins un métal additionnel choisi parmi le platine, le palladium, le rhénium, le ruthénium, le manganèse et le tantale et de manière très préférée choisi parmi le platine, le ruthénium et le rhénium. Le(s)dit(s) métal(ux) additionnel(s) est(sont) préférentiellement présent(s) avec une teneur présentant de 0,01 à 2 % poids, de préférence de 0,03 à 0,5 % poids par rapport au poids du catalyseur.

Préalablement à la mise en oeuvre du procédé selon l'invention, le catalyseur se trouve préférentiellement sous forme oxyde. Il présente des cristallites d'oxyde dudit métal du groupe VIII, présent dans la phase active dudit catalyseur, préférentiellement des cristallites d'oxyde de cobalt Co₃O₄, avec une taille réduite, favorisant une dispersion optimale dudit métal du groupe VIII présent dans la phase active dudit catalyseur. De manière préférée, lesdites cristallites d'oxyde de métal du groupe VIII présentent un diamètre moyen d'au moins 6 nm. De manière préférée, le diamètre moyen desdites cristallites d'oxyde de métal du groupe VIII ne dépasse pas 50 nm, de manière très préférée, il ne dépasse pas 30 nm voire 20 nm et de manière encore plus préférée, il ne dépasse pas 15 nm. La taille moyenne (diamètre moyen) des cristallites d'au moins dudit métal du groupe VIII présent dans la phase active du catalyseur est un paramètre de caractérisation de la dispersion dudit métal du groupe VIII présent dans la phase active dudit catalyseur, préférentiellement du cobalt. La taille moyenne des cristallites d'au moins dudit métal du groupe VIII présent dans la phase active du catalyseur est déterminée par diffraction des rayons X à partir de l'équation de Scherrer sur le pic de diffraction attribué audit métal du groupe VIII. Dans le cas préféré où ledit métal du groupe VIII est le cobalt, la taille moyenne des cristallites d'oxyde de cobalt est déterminée par diffraction des rayons X à partir de l'équation de Scherrer sur le pic de diffraction à 59,5° (plan 511) (raie principale de diffraction de Co₃O₄). La dispersion dudit métal du groupe VIII, présent dans la phase active du catalyseur, en particulier la dispersion du cobalt, peut également être estimée par une méthode de chimisorption du propène telle qu'elle est décrite dans la publication de A.S. Lermontov, J.S. Girardon, A. Griboval-Constant, S. Pietrzyk et A.Y. Khodakov, Catalysis Letters, 101 (2005) 117.

Le support sur lequel est déposée ladite phase active est avantageusement formé d'au moins un oxyde simple choisi parmi l'alumine (Al₂O₃), la silice (SiO₂), l'oxyde de titane (TiO₂), la cérine (CeO₂) et la zircone (ZrO₂). Il peut également être avantageusement formé de plusieurs oxydes simples choisis parmi l'alumine (Al₂O₃), la silice (SiO₂), l'oxyde de titane (TiO₂), la cérine (CeO₂) et la zircone (ZrO₂) et de manière très préférée ledit support est formé de silice et d'alumine. La phase alumine présente dans un support constitué de silice-alumine est une alumine de transition formée d'au moins une phase de structure cristallographique δ, γ, θ ou encore α. Un tel support constitué de silice-alumine comprend préférentiellement de 1 à 30% poids de silice. Ladite silice-alumine est homogène à l'échelle du micromètre, et de manière encore plus préférée, homogène à l'échelle du nanomètre. Le support sur lequel est déposée ladite phase active peut encore avantageusement être formé d'une spinelle incluse dans une alumine ou une silice-alumine, de préférence dans une silice-alumine. En particulier, ledit support du catalyseur peut être avantageusement constitué d'une spinelle simple, incluse dans une silice-alumine, de type MAl₂O₄/Al₂O₃.SiO₂ ou d'une spinelle mixte, incluse dans une silice-alumine, de type MₓM'₍₁₋ₓ₎Al₂O₄/Al₂O₃-SiO₂ où M et M' sont des métaux distincts choisis dans le groupe constitué par le magnésium (Mg), le cuivre (Cu), le cobalt (Co), le nickel (Ni), l'étain (Sn), le zinc (Zn), le lithium (Li), le calcium (Ca), le césium (Cs), le sodium (Na), le fer (Fe) et le manganèse (Mn), où Al₂O₃.SiO₂ désigne la formule chimique d'une silice-alumine, où x est compris entre 0 et 1, les valeurs 0 et 1 étant elles-mêmes exclues. Un tel support formé d'une structure spinelle comprend au moins 5% poids de ladite structure spinelle, de manière préférée au moins 10% poids, et de manière encore plus préférée au moins 15 % poids. La silice-alumine dans laquelle est préférentiellement incluse la structure spinelle comprend préférentiellement de 1 à 30% poids de silice. Elle est homogène à l'échelle du micromètre, et de manière encore plus préférée, homogène à l'échelle du nanomètre.

Un support formé d'une spinelle incluse dans une alumine ou une silice-alumine peut être préparé par toute méthode connue de l'Homme du métier. En particulier, tout procédé permettant d'obtenir ledit support, modifié par l'addition d'au moins un élément M pour obtenir une structure spinelle simple et d'au moins un élément M' pour obtenir une structure spinelle mixte, convient. Une méthode de préparation d'un tel support consiste, par exemple, à imprégner un support alumine ou silice-alumine, préformé ou en poudre, avec au moins une solution aqueuse contenant les précurseurs hydrosolubles des éléments choisis pour la structure spinelle et à procéder généralement à des étapes de lavage, séchage et enfin de calcination. Une autre méthode consiste à préparer ledit support du catalyseur, à base d'alumine ou de silice-alumine, par coprécipitation d'une solution aqueuse contenant les métaux Al, M et éventuellement M', par exemple sous forme de nitrates, par une solution aqueuse de carbonate ou d'hydrogénocarbonate alcalin, suivie d'un lavage, d'un séchage, et enfin d'une calcination. Une autre méthode consiste encore à préparer ledit support du catalyseur, à base d'alumine ou de silice-alumine, par le procédé sol-gel, ou encore par complexation d'une solution aqueuse contenant les métaux Al, M et éventuellement M', par au moins un acide alpha alcool ajouté à raison de 0,5 à 2 moles d'acide par mole de métaux, suivi d'un séchage sous vide conduisant à l'obtention d'une substance vitreuse homogène, puis d'une calcination. Ces méthodes de préparation d'un tel support pour l'obtention d'une spinelle incluse dans une alumine ou une silice-alumine sont bien connues de l'Homme du métier. Un tel support est soumis à un traitement thermique avant la préparation proprement dite du catalyseur utilisé dans le procédé selon l'invention afin d'obtenir la structure spinelle (aluminate de M ou aluminate de M et M', où M et M' ont les définitions données ci-dessus). Ledit traitement thermique est préférentiellement mis en oeuvre à une température comprise entre 600 et 1200°C, très préférentiellement entre 740 et 1030°C et de manière encore plus préférée entre 800 et 980°C. Il est effectué sous atmosphère oxydante, par exemple sous air ou sous air appauvri en oxygène. Il peut également être effectué au moins en partie sous azote.

Le support sur lequel est déposée ladite phase active peut présenter une morphologie sous forme de billes, d'extrudés (par exemple de forme trilobes) ou de pastilles, notamment lorsque ledit catalyseur est mis en oeuvre dans un réacteur fonctionnant en lit fixe, ou présenter une morphologie sous forme de poudre de granulométrie variable, notamment lorsque ledit catalyseur est mis en oeuvre dans un réacteur de type colonne à bulles (ou "slurry bubble column" selon la terminaison anglaise). La taille des grains du catalyseur peut être comprise entre quelques microns et quelques centaines de microns. Pour une mise en oeuvre en réacteur "slurry ", la taille des particules du catalyseur est préférentiellement comprise entre 10 microns et 500 microns, de manière préférée entre 10 microns et 300 microns, de manière très préférée entre 20 et 200 microns, et de manière encore plus préférée entre 30 et 160 microns.

Le catalyseur mis en oeuvre dans le procédé de synthèse d'hydrocarbures selon l'invention est préparé selon un procédé comprenant au moins :
i) au moins une étape de mise en contact d'au moins dudit support tel que décrit ci-dessus avec au moins une solution contenant au moins un précurseur dudit métal du groupe VIII,
ii) au moins une étape de mise en contact d'au moins dudit support tel que décrit ci-dessus avec au moins un composé organique formé d'au moins un oligosaccharide cyclique composé d'au moins 6 sous-unités glucopyranose liées en α-(1,4),
iii) au moins une étape de calcination pour obtenir au moins ledit métal dudit groupe VIII sous forme oxyde,
les étapes i) et ii) pouvant être réalisées séparément, dans un ordre indifférent, ou simultanément.

Le dépôt d'au moins dudit métal du groupe VIII sur ledit support, conformément à la mise en oeuvre de ladite étape i), peut être réalisé par toute méthode bien connue de l'Homme du métier. Ladite étape i) est préférentiellement réalisée par imprégnation du support par au moins une solution contenant au moins un précurseur dudit métal du groupe VIII. En particulier, ladite étape i) peut être réalisée par imprégnation à sec, par imprégnation en excès, ou encore par dépôt - précipitation (tel que décrit dans les brevets US 5.874.381 et US 6.534.436) selon des méthodes bien connues de l'Homme du métier. De manière préférée, ladite étape i) est réalisée par imprégnation à sec, laquelle consiste à mettre en contact le support du catalyseur avec une solution, contenant au moins un précurseur dudit métal du groupe VIII, dont le volume est égal au volume poreux du support à imprégner. Cette solution contient les précurseurs métalliques du ou des métaux du groupe VIII à la concentration voulue.

Le(s)dit(s) métal(ux) du groupe VIII est(sont) mis au contact dudit support par l'intermédiaire de tout précurseur métallique soluble en phase aqueuse ou en phase organique. Lorsqu'il est introduit en solution organique, ledit précurseur du métal du groupe VIII est par exemple l'oxalate ou l'acétate dudit métal du groupe VIII. De manière préférée, ledit précurseur du métal du groupe VIII est introduit en solution aqueuse, par exemple sous forme de nitrate, de carbonate, d'acétate, de chlorure, d'oxalate, de complexes formés par un polyacide ou un acide-alcool et ses sels, de complexes formés avec les acétylacétonates, ou de tout autre dérivé inorganique soluble en solution aqueuse, laquelle est mise en contact avec ledit support. Dans le cas préféré où ledit métal du groupe VIII est le cobalt, on utilise avantageusement comme précurseur de cobalt, le nitrate de cobalt, l'oxalate de cobalt ou l'acétate de cobalt.

La mise en contact dudit composé organique employé pour la mise en oeuvre de ladite étape ii) avec ledit support est réalisée par imprégnation, notamment par imprégnation à sec ou imprégnation en excès, préférentiellement par imprégnation à sec. Ledit composé organique est préférentiellement imprégné sur ledit support après solubilisation en solution aqueuse.

Ledit composé organique est formé d'au moins un oligosaccharide cyclique composé d'au moins 6 sous-unités glucopyranose liées en α-(1,4). Une représentation spatiale d'une sous-unité glucopyranose est donnée ci-dessous :

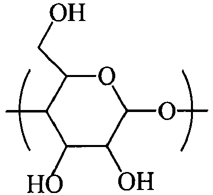

Ledit composé organique est préférentiellement choisi parmi les cyclodextrines, les cyclodextrines substituées, les cyclodextrines polymérisées et les mélanges de cyclodextrines. Les cyclodextrines sont une famille d'oligosaccharides cycliques composés de sous-unités glucopyranose liées en α-(1,4). Il s'agit de molécules-cages. Selon l'invention, les cyclodextrines préférées sont l'α-cyclodextrine, la β-cyclodextrine et la γ-cyclodextrine respectivement composée de 6, 7 et 8 sous-unités glucopyranose liées en α-(1,4). Les représentations développées de l'α-cyclodextrine, de la β-cyclodextrine et de la γ-cyclodextrine sont données ci-dessous. On utilise préférentiellement pour la mise en oeuvre de ladite étape ii) la β-cyclodextrine composée de 7 sous-unités glucopyranose liées en α-(1,4). Les cyclodextrines sont des composés commercialisés.

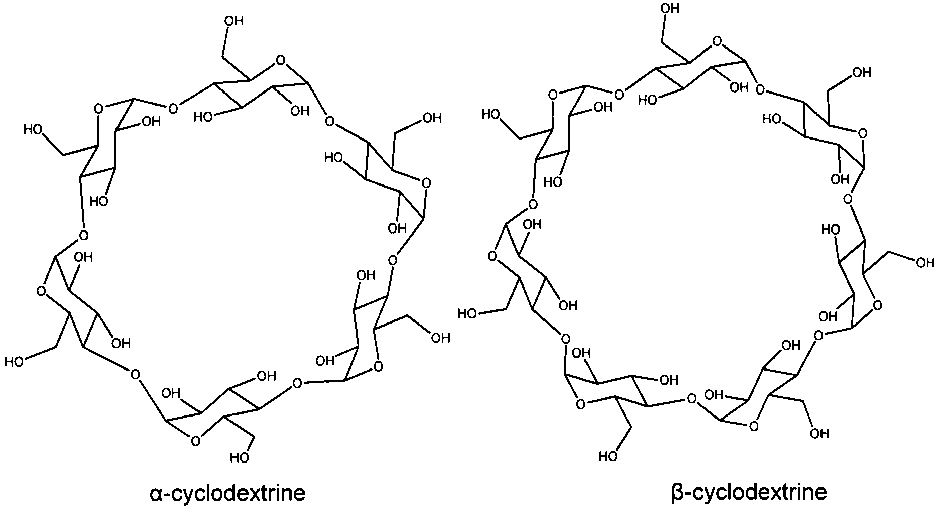

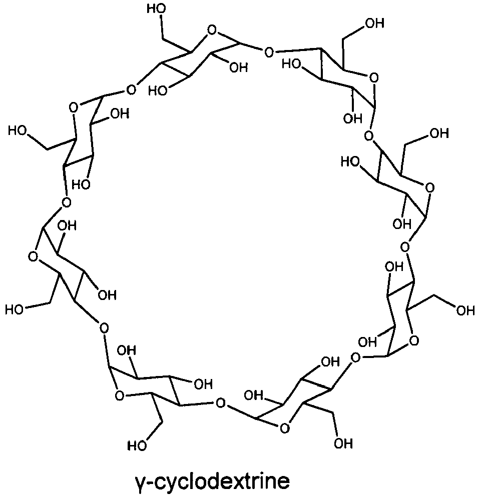

Les cyclodextrines substituées avantageusement employées pour la mise en oeuvre de ladite étape ii) sont constituées de 6, 7 ou 8 sous-unités glucopyranose liées en α-(1,4), dont l'une au moins est mono- ou polysubstituée. Les substituants peuvent être fixés sur un ou plusieurs groupe(s) hydroxyle(s) présent(s) dans la molécule, à savoir sur les groupes hydroxyles directement liés au cycle d'une unité glucopyranose et/ou sur l'hydroxyle lié au groupement CH₂ lui-même lié au cycle d'une unité glucopyranose. De manière plus préférée, lesdites cyclodextrines substituées portent un ou plusieurs substituant(s), identique(s) ou différent(s), choisi(s) parmi les radicaux alkyles saturés ou non, fonctionnalisés ou non, et les fonctions esters, carbonyles, carboxyles, carboxylates, phosphates, éthers, polyéthers, urées, amide, amino, triazole, ammonium. Les cyclodextrines substituées préférées sont les cyclodextrines méthylées, éthylées, propylées, allylées (c'est-à-dire possédant une fonction ayant pour formule semi-développée -CH₂-CH=CH₂), succinylées (c'est-à-dire possédant une fonction ayant pour formule semi-développée R-OCO-CH₂-CH₂COOH), carboxylées, carboxyméthylées, acétylées, 2-hydroxypropylées et polyoxyéthylénées. Les groupements mono- ou polysubstituants de la cyclodextrine peuvent aussi être une molécule de monosaccharide ou disaccharide telle qu'une molécule de maltose, glucose, fructose ou saccharose.

Les cyclodextrines substituées particulièrement avantageuses pour la mise en oeuvre de ladite étape ii) sont l'hydroxypropyl bêta-cyclodextrine et les bêta-cyclodextrines méthylées.

Les cyclodextrines polymérisées avantageusement employées pour la mise en oeuvre de ladite étape ii) sont des polymères dont les monomères sont chacun constitués d'un oligosaccharide cyclique composé de 6, 7 ou 8 sous-unités glucopyranose liées en α-(1,4), substituées ou non. Une cyclodextrine sous forme polymérisée, réticulée ou non, pouvant être avantageusement utilisée pour la mise en oeuvre de ladite étape ii) est par exemple du type de celle obtenue par polymérisation de monomères de bêta-cyclodextrine avec de l'épichlorhydrine ou un polyacide.

Les mélanges de cyclodextrines avantageusement employés pour la mise en oeuvre de ladite étape ii) mettent en oeuvre des cyclodextrines substituées ou non. Lesdits mélanges pourront, à titre d'exemple, contenir conjointement et dans des proportions variables, chacun des trois types de cyclodextrines (alpha, bêta et gamma).

L'introduction dudit composé organique, préférentiellement d'une cyclodextrine et très préférentiellement de la bêta-cyclodextrine, pour la mise en oeuvre de ladite étape ii) est telle que le rapport molaire {(métal(ux) du groupe VIII sous forme d'oxyde présent dans phase active du catalyseur obtenu à l'issue de ladite étape iii)/composé organique} est compris entre 10 et 300 et de préférence entre 50 et 180. Le(s) métal(ux) du groupe VIII pris en compte pour le calcul dudit rapport molaire sont le(s) métal(ux) introduit(s) pour la mise en oeuvre de ladite étape i) et se trouvant sous la forme d'oxyde dans la phase active du catalyseur issu de ladite étape iii). Le(s)dit(s) métal(ux) du groupe VIII est(sont) en conséquence réductible(s) : il(s) sera(ont) réduit(s) préalablement à la mise en oeuvre du procédé de synthèse d'hydrocarbures selon l'invention. Il n'est pas tenu compte, en particulier, pour le calcul dudit rapport molaire de la quantité de métal de groupe VIII éventuellement présente dans une structure spinelle (métal M ou M' selon la définition donnée plus haut dans la présente description) employée comme composant du support du catalyseur.

Le procédé de préparation du catalyseur utilisé dans le procédé de synthèse d'hydrocarbures selon l'invention comporte plusieurs modes de mises en oeuvre.

Un premier mode de mise en oeuvre consiste à effectuer lesdites étapes i) et ii) de façon simultanée de sorte que ledit composé organique, de préférence une cyclodextrine, et au moins ledit précurseur d'au moins dudit métal du groupe VIII présent dans la phase active sont co-imprégnés sur ledit support (étape de co-imprégnation). Ledit premier mode de mise en oeuvre comprend avantageusement la mise en oeuvre d'une ou plusieurs étapes i). En particulier, une ou plusieurs étapes i) précède(nt) et/ou suive(nt) avantageusement ladite étape de co-imprégnation. Conformément audit premier mode de mise en oeuvre, chacune des étapes réalisées est préférentiellement immédiatement suivie d'au moins une étape de séchage puis d'au moins une étape de calcination. En particulier, ladite étape de co-imprégnation est suivie d'au moins une étape de séchage puis d'au moins une étape de calcination. Ledit premier mode de mise en oeuvre peut comprendre plusieurs étapes de co-imprégnation. Ladite étape iii) de calcination est au moins réalisée lorsque toutes les étapes de dépôt d'au moins dudit métal du groupe VIII sur le support du catalyseur ont été effectuées.

Un deuxième mode de mise en oeuvre consiste à effectuer ladite étape i) préalablement à ladite étape ii). Conformément audit deuxième mode de mise en oeuvre, une ou plusieurs étapes i) de dépôt d'au moins dudit métal du groupe VIII présent dans la phase active du catalyseur précède(nt) ladite étape ii). De préférence, chacune desdites étapes i) est immédiatement suivie d'au moins une étape de séchage et d'au moins une étape de calcination. En particulier, la dernière étape i) est avantageusement suivie d'au moins une étape de séchage et d'au moins une étape de calcination conformément à ladite étape iii) avant la mise en oeuvre de ladite étape ii). Ladite étape ii) est avantageusement suivie d'au moins une étape de séchage et éventuellement d'au moins une étape de calcination.

Un troisième mode de mise en oeuvre consiste à effectuer ladite étape ii) préalablement à ladite étape i). Ladite étape ii) est préférentiellement immédiatement suivie d'au moins une étape de séchage et éventuellement d'au moins une étape de calcination avant la mise en oeuvre de ladite étape i). De manière avantageuse, ladite étape ii) est suivie de plusieurs étapes i). La préparation du catalyseur conformément audit troisième mode de réalisation se termine avantageusement par ladite étape iii) de calcination.

Chacun des trois modes de mises en oeuvre décrits ci-dessus peut être effectué de manière indépendante de sorte que le catalyseur utilisé dans le procédé selon l'invention est préparé soit selon ledit premier mode de mise en oeuvre, soit selon ledit deuxième mode de mise en oeuvre soit encore selon ledit troisième mode de mise en oeuvre. Toutefois, il peut être avantageux d'associer ledit premier mode avec ledit deuxième mode ou avec ledit troisième mode : aussi bien le métal du groupe VIII présent dans la phase active que le composé organique, préférentiellement une cyclodextrine, sont déposés au moins à deux reprises sur le support du catalyseur, à savoir au moins une fois par co-imprégnation et au moins une fois par imprégnation successive.

Les étapes de séchage, mises en oeuvre pour la préparation du catalyseur, préparé selon au moins un mode de mise en oeuvre décrit ci-dessus, sont réalisées à une température comprise entre 80 et 160°C. Elles sont préférentiellement réalisées pendant une durée comprise entre 1 et 4 heures. Ladite étape iii) de calcination est réalisée à une température comprise entre 200 et 800°C, de préférence entre 250 et 600°C et de manière très préférée entre 300 et 430°C. Elle est préférentiellement réalisée pendant une durée comprise entre 2 et 6 heures. Les étapes de calcination, mises en oeuvre pour la préparation du catalyseur, préparé selon au moins un mode de mise en oeuvre décrit ci-dessus, sont avantageusement réalisées dans les mêmes conditions que ladite étape iii).

La préparation du catalyseur, utilisé dans le procédé de synthèse d'hydrocarbures selon l'invention, comprend avantageusement au moins une étape iv) consistant à déposer au moins un métal additionnel choisi parmi le platine, le palladium, le rhénium, le rhodium, le ruthénium, le manganèse et le tantale sur ledit support du catalyseur. De manière préférée, ledit métal additionnel est choisi parmi le platine, le ruthénium et le rhénium et de manière très préférée, ledit métal additionnel est le platine. Le dépôt d'au moins dudit métal additionnel sur ledit support peut être réalisé par toute méthode connue de l'Homme du métier, préférentiellement par imprégnation du support du catalyseur par au moins une solution contenant au moins un précurseur dudit métal additionnel, par exemple par imprégnation à sec ou par imprégnation en excès. Ladite étape iv) peut être réalisée soit séparément des étapes i) et ii), dans un ordre indifférent, soit simultanément avec ladite étape i) et/ou ladite étape ii). Plus précisément, elle peut être réalisée en association avec au moins l'un des trois modes de mise en oeuvre de préparation du catalyseur décrits ci-dessus. De manière préférée, ladite étape iv) de dépôt d'au moins dudit métal additionnel est réalisée simultanément avec au moins une étape i) de dépôt d'au moins dudit métal du groupe VIII. Selon un mode de réalisation très préféré, le catalyseur est préparé selon un procédé comprenant au moins une étape de co-imprégnation dudit métal du groupe VIII et dudit composé organique, de préférence une cyclodextrine et très préférentiellement la bêta-cyclodextrine, et au moins une étape de co-imprégnation d'au moins dudit métal du groupe VIII et d'au moins dudit métal additionnel. Ladite étape iv) est préférentiellement immédiatement suivie d'au moins une étape de séchage puis d'au moins une étape de calcination dans des conditions (température, durée) telles que celles décrites ci-dessus.

Le catalyseur, préparé selon au moins l'un des modes de mises en oeuvre décrits ci-dessus, se trouve, avant la mise en oeuvre du procédé de synthèse d'hydrocarbures selon l'invention, sous forme oxyde : le(s) métal(ux) du groupe VIII présent(s) dans la phase active sont à l'état oxyde et se présentent sous forme de cristallites d'oxyde dudit métal du groupe VIII de taille réduite. Ledit catalyseur peut être entièrement ou partiellement débarrassé dudit composé organique formé d'au moins un oligosaccharide cyclique composé d'au moins 6 sous-unités glucopyranose liées en α-(1,4).

Préalablement à son utilisation dans le réacteur catalytique et la mise en oeuvre du procédé selon l'invention, le catalyseur subit au moins un traitement réducteur, par exemple avec de l'hydrogène, pur ou dilué, à haute température. Ce traitement permet d'activer ledit catalyseur et de former des particules de métal, en particulier de métal du groupe VIII, à l'état zéro valent. La température de ce traitement réducteur est préférentiellement comprise entre 200 et 500 °C et sa durée est comprise entre 2 et 20 heures.

Ce traitement réducteur est effectué soit *in situ* (dans le même réacteur que celui où est opérée la réaction de Fischer-Tropsch selon le procédé de l'invention), soit *ex situ* avant d'être chargé dans le réacteur.

Le procédé de synthèse d'hydrocarbures selon l'invention est opéré sous une pression totale comprise entre 0,1 et 15 MPa, de préférence entre 0,5 et 10MPa, sous une température comprise entre 150 et 350°C, de préférence entre 180 et 270°C. La vitesse volumique horaire est avantageusement comprise entre 100 et 20000 volumes de gaz de synthèse par volume de catalyseur et par heure (100 à 20000 h⁻¹) et de préférence entre 400 et 10000 volumes de gaz de synthèse par volume de catalyseur et par heure (400 à 10000 h⁻¹).

L'invention est illustrée par les exemples qui suivent.

### Exemples

L'ensemble des catalyseurs préparés dans les exemples 1 et 2 sont préparés à iso-teneur en cobalt réductible, c'est-à-dire en cobalt sous forme oxyde pouvant être réduit en cobalt métallique par un traitement réducteur à une température inférieure à 500°C.

### Exemple 1 (comparatif) : préparation de catalyseurs en l'absence d'oligosaccharide cyclique

### Exemple 1.1 : Préparation du catalyseur A de formule Co/Al₂O₃

Un catalyseur A1 comprenant du cobalt déposé sur un support d'alumine est préparé par imprégnation à sec d'une solution aqueuse de nitrate de cobalt de manière à déposer en deux étapes successives de l'ordre de 13,5% poids de Co sur une poudre d'alumine gamma (PURALOX SCCa 5/170, SASOL) de granulométrie moyenne égale à 80 µm, de surface 165 m²/g et de volume poreux égal 0,477 ml/g.

Après une première imprégnation à sec, le solide est séché en lit traversé à 120°C pendant 3h sous air puis calciné à 400°C pendant 4h en lit traversé sous flux d'air. Le catalyseur intermédiaire contient environ 8% poids de Co. Il est soumis à une deuxième étape d'imprégnation à sec au moyen d'une solution de nitrate de cobalt. Le solide obtenu est séché en lit traversé à 120°C pendant 3h sous air puis calciné à 400°C pendant 4h en lit traversé sous flux d'air. On obtient le catalyseur final A1 qui contient 13,5% poids de Co (cobalt réductible).

### Exemple 1.2 : Préparation du catalyseur A2 de formule Co/Al₂O₃ en présence de saccharose

Le saccharose ou sucrose est un sucre double formé par la condensation de 2 oses : une molécule de glucose et une molécule de fructose. Il ne s'agit pas d'un oligosaccharide cyclique. La formule développée du saccharose est donnée ci-dessous.

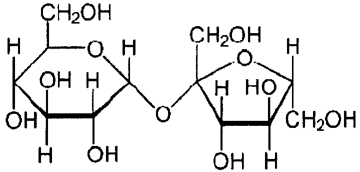

Le support du catalyseur A2 est identique à celui du catalyseur A1 décrit dans l'exemple 1.1.

Dans une première étape, on imprègne une solution aqueuse de nitrate de cobalt sur ledit support. Le solide obtenu est séché en lit traversé à 120°C pendant 3 heures sous air puis calciné à 400°C pendant 4 heures en lit traversé pour obtenir un catalyseur intermédiaire contenant au total une teneur de 7,6% poids de Co. Dans une deuxième étape, ce catalyseur intermédiaire est imprégné par une solution aqueuse contenant du nitrate de cobalt et du saccharose (fourni par Sigma-Aldrich, 99% de pureté, 44 mg de saccharose par gramme de catalyseur A2 préparé). Le solide obtenu est séché à 120°C pendant 3 heures puis calciné à 400°C pendant 4 heures. La concentration en Co dans chacune des solutions de nitrate de cobalt est ajustée pour obtenir le catalyseur A2 avec la teneur finale en Co voulue. Le catalyseur final A2 présente une teneur totale en cobalt de 13,7% poids (cobalt réductible).

### Exemple 1.3 : Préparation du catalyseur B1 de formule Co / Al₂O₃.SiO₂

Un catalyseur B1 comprenant du cobalt déposé sur un support de silice-alumine est préparé par imprégnation à sec d'une solution aqueuse de nitrate de cobalt de manière à déposer en deux étapes successives environ 14% poids de Co sur une silice-alumine contenant initialement 5% poids de SiO₂ et possédant une surface spécifique de 173 m²/g et un volume poreux de 0,55 ml/g (Siralox 5/170, SASOL).

Après une première imprégnation à sec, le solide est séché en lit traversé à 120°C pendant 3h sous air puis calciné à 400°C pendant 4h en lit traversé. Le catalyseur intermédiaire contient environ 8% poids de Co. Il est soumis à une deuxième étape d'imprégnation à sec au moyen d'une solution de nitrate de cobalt. Le solide obtenu est séché en lit traversé à 120°C pendant 3h sous air puis calciné à 400°C pendant 4h en lit traversé. On obtient le catalyseur final B1 qui contient 13,9% poids de Co (cobalt réductible).

### Exemple 1.4 : Préparation du catalyseur B2 de formule Co / CoAl₂O₄-Al₂O₃.SiO₂

Un catalyseur B2 comprenant du cobalt déposé sur un support, à base d'une spinelle incluse dans une silice-alumine, est préparé par imprégnation à sec d'une solution aqueuse de nitrate de cobalt de manière à déposer en deux étapes successives environ 13,7 % poids de cobalt sur le support.

La spinelle présente dans le support du catalyseur B2 est une spinelle simple formée d'aluminate de cobalt, lequel est inclus dans une silice-alumine contenant 5% poids de SiO₂, et présentant une surface spécifique de 173 m²/g et un volume poreux de 0,55 ml/g (Siralox 5/170, SASOL). La préparation de la spinelle incluse dans la silice-alumine est effectuée par imprégnation à sec d'une solution aqueuse de nitrate de cobalt de manière à introduire 5% poids de Co dans ladite silice-alumine. Après séchage à 120°C pendant 3 heures, le solide est calciné à 850°C pendant 4 heures sous air. Le support du catalyseur B2 est formé de 5% poids de cobalt sous forme d'aluminate de cobalt (soit 15% poids de spinelle) dans la silice-alumine.

La phase active à base de cobalt est ensuite déposée sur ledit support en deux étapes successives, par imprégnation à sec, selon un protocole identique à celui décrit pour la préparation du catalyseur A1. Les étapes de séchage et de calcination sont également réalisées dans les mêmes conditions opératoires que celles de l'exemple 1.1. La concentration en cobalt dans la solution de nitrate de cobalt, utilisée pour les imprégnations successives, est choisie pour obtenir le catalyseur B2 avec la teneur finale en Co voulue.

Le catalyseur B2 final présente une teneur totale en cobalt de 18,7% poids (la teneur en Co présent dans la phase spinelle étant comprise) et une teneur en cobalt réductible de 13,7 % poids.

### Exemple 2 (invention) : préparation de catalyseurs en présence d'oligosaccharide cyclique Cevelodextrine)

### Exemple 2.1 : Préparation du catalyseur C de formule Co/CoAl₂O₄-Al₂O₃.SiO₂

Le support du catalyseur C est identique à celui du catalyseur B2 décrit dans l'exemple 1.4. Il est préparé d'une manière analogue à celle indiquée dans l'exemple 1.4.

Dans une première étape, une solution contenant de la β-cyclodextrine (SIGMA-ALDRICH, pureté de 98%, 39 mg de β-cyclodextrine par gramme de catalyseur C préparé) est déposée par imprégnation à sec sur ledit support. Le solide est ensuite séché sous air en réacteur de type lit traversé durant 3 heures à 120°C. Le dépôt de la phase active à base de Co est ensuite réalisé en deux étapes successives, par imprégnation à sec, suivant le même protocole que pour le catalyseur B2. La concentration en cobalt dans la solution de nitrate de cobalt, utilisée pour les imprégnations successives, est choisie pour obtenir le catalyseur C avec la teneur finale en Co voulue.

Le catalyseur final C présente une teneur totale en cobalt de 18,8% poids (la teneur en Co présent dans la phase spinelle étant comprise) et une teneur en cobalt réductible de 13,7 % poids.

### Exemple 2.2 : Préparation du catalyseur D de formule Co/CoAl₂O₄-Al₂O₃.SiO₂

Le support du catalyseur D est identique à celui du catalyseur B2 décrit dans l'exemple 1.4. Il est préparé d'une manière analogue à celle indiquée dans l'exemple 1.4.

Dans une première étape, on imprègne une solution aqueuse contenant du nitrate de cobalt et de la β-cyclodextrine (SIGMA-ALDRICH, pureté de 98%, 26mg de β-cyclodextrine par gramme de catalyseur D préparé) sur ledit support (étape de co-imprégnation). Le solide obtenu est séché en lit traversé à 120°C pendant 3 heures sous air puis calciné à 400°C pendant 4 heures en lit traversé pour obtenir un catalyseur intermédiaire contenant au total 13% poids de Co (teneur en Co présent dans la phase spinelle comprise). Il est soumis à une deuxième étape d'imprégnation à sec au moyen d'une solution de nitrate de cobalt. Le solide obtenu est séché en lit traversé à 120°C pendant 3h sous air puis calciné à 400°C pendant 4h en lit traversé. La concentration en Co dans chacune des solutions de nitrate de cobalt est choisie pour obtenir le catalyseur D avec la teneur finale en Co voulue.

Le catalyseur final D présente une teneur totale en cobalt de 18,9% poids (teneur en Co présent dans la phase spinelle comprise) et une teneur en cobalt réductible de 13,7 % poids.

### Exemple 2.3 : Préparation du catalyseur E de formule Co/CoAl₂O₄-Al₂O₃.SiO₂

Le support du catalyseur E est identique à celui du catalyseur B2 décrit dans l'exemple 1.4. Il est préparé d'une manière analogue à celle indiquée dans l'exemple 1.4.

Dans une première étape, on imprègne une solution aqueuse de nitrate de cobalt sur ledit support. Le solide obtenu est séché en lit traversé à 120°C pendant 3 heures sous air puis calciné à 400°C pendant 4 heures en lit traversé pour obtenir un catalyseur intermédiaire contenant au total une teneur de 12,9% poids de Co (teneur en Co présent dans la phase spinelle comprise). Dans une deuxième étape, ce catalyseur intermédiaire est imprégné par une solution aqueuse contenant du nitrate de cobalt et de la β-cyclodextrine (SIGMA-ALDRICH, pureté de 98%, 37mg de β-cyclodextrine par gramme de catalyseur E préparé). Le solide obtenu est séché à 120°C pendant 3 heures puis calciné à 400°C pendant 4 heures. La concentration en Co dans chacune des solutions de nitrate de cobalt est choisie pour obtenir le catalyseur E avec la teneur finale en Co voulue.

Le catalyseur final E présente une teneur totale en cobalt de 19,0% poids (la teneur en Co présent dans la phase spinelle étant comprise) et une teneur en cobalt réductible de 13,9 % poids.

### Exemple 2.4 : Préparation du catalyseur F de formule Co/CoAl₂O₄-Al₂O₃.SiO₂

Le support du catalyseur F est identique à celui du catalyseur B2 décrit dans l'exemple 1.4. Il est préparé d'une manière analogue à celle indiquée dans l'exemple 1.4.

Une solution contenant de la β-cyclodextrine (SIGMA-ALDRICH, pureté de 98%, 37mg de β-cyclodextrine par gramme de catalyseur F préparé) est déposée en post-traitement sur un catalyseur préparé dans les mêmes conditions que le catalyseur B2. Le solide obtenu a ensuite été séché en réacteur de type lit traversé sous flux d'air durant 3 heures à 120°C.

Le catalyseur final F présente une teneur totale en cobalt de 18,7% poids (teneur en Co présent dans la phase spinelle comprise) et une teneur en cobalt réductible de 13,7 % poids.

### Exemple 2.5 : Préparation du catalyseur G de formule Co(Pt)/CoAl₂O₄-Al₂O₃.SiO₂

Le support du catalyseur G est identique à celui du catalyseur B2 décrit dans l'exemple 1.4. Il est préparé d'une manière analogue à celle indiquée dans l'exemple 1.4.

Dans une première étape, on imprègne une solution aqueuse contenant du nitrate de cobalt et de la β-cyclodextrine (SIGMA-ALDRICH, pureté de 98%, 26mg de β-cyclodextrine par gramme de catalyseur G préparé) sur ledit support (étape de co-imprégnation). Le solide obtenu est séché en lit traversé à 120°C pendant 3 heures sous air puis calciné à 400°C pendant 4 heures en lit traversé pour obtenir un catalyseur intermédiaire contenant au total 13% poids de Co (teneur en Co présent dans la phase spinelle comprise). Il est soumis à une deuxième étape de co-imprégnation au moyen d'une solution aqueuse contenant du nitrate de cobalt et de l'hydroxyde de tétrammineplatine de formule [Pt(NH₃)₄](OH)₂. Le solide obtenu est séché à 120°C pendant 3h sous air sec puis calciné à 400°C pendant 4h sous air sec. La concentration en Co dans chacune des solutions de nitrate de cobalt et celle en platine dans la solution d'hydroxyde de tétrammineplatine sont choisies pour obtenir le catalyseur G avec la teneur finale en Co et Pt voulue.

Le catalyseur final G présente une teneur en platine de 0,0545% poids, une teneur totale en cobalt de 18,9% poids (la teneur en Co présent dans la phase spinelle étant comprise) et une teneur en cobalt réductible de 13,8 % poids.

### Exemple 2.6 : Préparation du catalyseur H de formule Co/Al₂O₃

Le support du catalyseur H est identique à celui du catalyseur A1.

Dans une première étape, on imprègne une solution aqueuse de nitrate de cobalt sur ledit support. Le solide obtenu est séché en lit traversé à 120°C pendant 3 heures sous air puis calciné à 400°C pendant 4 heures en lit traversé pour obtenir un catalyseur intermédiaire contenant au total une teneur de 7,5% poids de Co. Dans une deuxième étape, ce catalyseur intermédiaire est imprégné par une solution aqueuse contenant du nitrate de cobalt et de la β-cyclodextrine (fourni par SIGMA-ALDRICH, 98% de pureté), 24mg de β-cyclodextrine par gramme de catalyseur H préparé). Le solide obtenu est séché à 120°C pendant 3 heures puis calciné à 400°C pendant 4 heures. La concentration en Co dans chacune des solutions de nitrate de cobalt est choisie pour obtenir le catalyseur H avec la teneur finale en Co voulue.

Le catalyseur final H présente une teneur totale en cobalt de 13,7% poids (Co réductible).

### Exemple 2.7 : Préparation d'un catalyseur I de formule Co/Al₂O₃.SiO₂

Le support du catalyseur I est identique à celui du catalyseur B1 décrit dans l'exemple 1.3. Dans une première étape, on imprègne une solution aqueuse contenant du nitrate de cobalt et de la β-cyclodextrine (SIGMA-ALDRICH, pureté de 98%, 26 mg de β-cyclodextrine par gramme de catalyseur I préparé) sur ledit support (étape de co-imprégnation). Le solide obtenu est séché en lit traversé à 120°C pendant 3 heures sous air puis calciné à 400°C pendant 4 heures en lit traversé pour obtenir un catalyseur intermédiaire contenant au total 6,7% poids de Co. Il est soumis à une deuxième étape d'imprégnation à sec au moyen d'une solution de nitrate de cobalt. Le solide obtenu est séché en lit traversé à 120°C pendant 3h sous air puis calciné à 400°C pendant 4h en lit traversé. La concentration en Co dans chacune des solutions de nitrate de cobalt est choisie pour obtenir le catalyseur I avec la teneur finale en Co voulue.

Le catalyseur final I présente une teneur totale en cobalt de 13,7% poids (Co réductible).

Le tableau 1 ci-dessous résume les teneurs totales en cobalt de chaque catalyseur final, les quantités introduites de composé organique par gramme de catalyseur (β-cyclodextrine ou saccharose), le rapport molaire Coₜₒₜₐₗ/composé organique et le rapport molaire Co_{réductible} (hors ₛₚᵢₙₑₗₗₑ₎/composé organique, le composé organique étant la β-cyclodextrine pour les catalyseurs C à I et le saccharose pour le catalyseur A2.

**Tableau 1 : teneur en Co et composé organique pour chaque catalyseur**

| Catalyseur | %poids total de cobalt | %poids de composé organique | Ratio molaire Coₜₒₜₐₗ/ composé organique (1) | Ratio molaire Co_{réductible (hors spinelle)}/ composé organique (2) |
|---|---|---|---|---|
| A1 (comparatif) | 13,5 | - | - | - |
| A2 (comparatif) | 13,7 | 4,4 | 18 | 18 |
| B1 (comparatif) | 13,9 | - | - | - |
| B2 (comparatif) | 18,7 | - | - | - |
| C (invention) | 18,8 | 3,9 | 92 | 69 |
| D (invention) | 18,9 | 2,6 | 139 | 104 |
| E (invention) | 19,0 | 3,7 | 106 | 79 |
| F (invention) | 18,7 | 3,7 | 105 | 78 |
| G (invention) | 18,9 | 2,6 | 139 | 104 |
| H (invention) | 13,7 | 2,4 | 108 | 108 |
| I (invention) | 13,7 | 2,6 | 103 | 103 |

| | | | | |
|---|---|---|---|---|
| (1) : la teneur en cobalt dont il est tenu compte pour le calcul du ratio molaire Coₜₒₜₐₗ/composé organique, en particulier du ratio molaire Coₜₒₜₐₗ/β**-**cyclodextrine, est celle résultant non seulement du cobalt présent dans la phase spinelle du support et également du cobalt, sous la forme d'oxyde, présent dans la phase active du catalyseur et introduit par différentes étapes d'imprégnations sur le support. (2) : la teneur en cobalt dont il est tenu compte pour le calcul du ratio molaire Co_{réductible} (hors ₛₚᵢₙₑₗₗₑ₎/composé organique, en particulier du ratio molaire Co_{réductible} (hors ₛₚᵢₙₑₗₗₑ₎/β-cyclodextrine, est celle liée au cobalt, sous la forme d'oxyde, présent dans la phase active du catalyseur et introduit par différentes étapes d'imprégnations sur le support. Il n'est pas tenu compte du cobalt présent dans la phase spinelle du support. | | | | |

### Exemple 3 : Dispersion du cobalt présent dans les catalyseurs comparatifs A1, A2, B1 et B2 et dans les catalyseurs conformes C, D, E, G, H et I.

Dans cet exemple, la dispersion du cobalt est caractérisée d'une part, par la détermination de la taille moyenne des cristallites d'oxyde de cobalt (Co₃O₄) présentes dans chacun des catalyseurs. La taille moyenne des cristallites d'oxyde de cobalt est déterminée par diffraction des rayons X à partir de l'équation de Scherrer sur le pic de diffraction à 59,5° (plan 511). Le diamètre des cristallites de Co₃O₄ est exprimé en nanomètre (nm). Pour chaque catalyseur, la taille des cristallites, exprimée par leur diamètre, figure dans le tableau 2.

La dispersion du cobalt est caractérisée, d'autre part, par une méthode complémentaire basée sur la chimisorption du propène laquelle est réalisée sous forme de pulse de propène de volume de 50 µL à 50°C sous flux d'hélium. Dans cette méthode, décrite plus en détail dans la publication de A.S. Lermontov, J.S. Girardon, A. Griboval-Constant, S. Pietrzyk et A.Y. Khodakov, Catalysis Letters, 101 (2005) 117, chaque catalyseur est préalablement réduit sous flux d'hydrogène à 400°C pendant 10 heures et avec une vitesse volumique horaire (wh) égale à 12500 h⁻¹. Les résultats sont exprimés en micromoles de propène chimisorbé par gramme de cobalt réductible du catalyseur. Pour chaque catalyseur, la quantité de propène chimisorbé est présentée dans le tableau 2.

**Tableau 2 : caractérisation de la dispersion du cobalt pour chaque catalyseur**

| Catalyseur | %poids total de cobalt | %poids de β-cyclodextrine ou saccharose | Taille des cristallites de Co₃O₄ (nm) | Propène chimisorbé (µmoles/g Co réductible) |
|---|---|---|---|---|
| A1 (comparatif) | 13,5 | 0,0 | 12,4 | 140,0 |
| A2 (comparatif) | 13,7 | 4,4 | 11,0 | 137,8 |
| B1 (comparatif) | 13,9 | 0,0 | 12,8 | 140,3 |
| B2 (comparatif) | 18,7 | 0,0 | 12,3 | 146,0 |
| C (invention) | 18,8 | 3,9 | 7,5 | 176,6 |
| D (invention) | 18,9 | 2,6 | 7,9 | 193,4 |
| E (invention) | 19,0 | 3,7 | 11,0 | 159,0 |
| G (invention) | 18,9 | 2,6 | 7,6 | 240,6 |
| H (invention) | 13,7 | 2,4 | 10,2 | 157,6 |
| I (invention) | 13,7 | 2,6 | 8,2 | 180,3 |

En comparant les catalyseurs ayant des supports identiques (H envers A1 et A2 ; I envers B1 ; C, D, E et G envers B2), les résultats montrent que les catalyseurs préparés en présence de cyclodextrine présentent une taille moyenne de cristallites de Co₃O₄ significativement plus petite que celle des catalyseurs préparés en l'absence de composé organique ou en présence d'un oligosaccharide non cyclique (saccharose employé pour le catalyseur A2). Cette taille varie en fonction du mode d'utilisation de la β-cyclodextrine et de la quantité introduite. Il apparaît également que la quantité de propène chimisorbé semble reliée de cette taille de cristallites. Les résultats figurant dans le tableau 2 montrent que plus la taille de cristallites est petite, plus la quantité de propène chimisorbé est importante, ce qui traduit une augmentation du nombre de sites actifs, en raison non seulement de l'augmentation du nombre de cristallites de Co₃O₄ mais également de la bonne réductibilité du cobalt. Les résultats figurant dans le tableau 2 démontrent que le catalyseur D, préparé par co-imprégnation de beta-cyclodextrine et de cobalt conduit à une très bonne dispersion de cobalt et une quantité de propène chimisorbé élevée. Le catalyseur G, également préparé par co-imprégnation de beta-cyclodextrine et de cobalt, et comprenant du platine dans sa composition conduit aux meilleurs résultats.

### Exemple 4 : Performances catalvtiaues des catalyseurs A1, A2, B1, B2, C, D, E, F, G, H et I en conversion du gaz de synthèse.

Les catalyseurs A1, A2, B1, B2, C, D, E, F, G, H et I, avant d'être successivement testés en conversion du gaz de synthèse, sont réduits *in situ* sous un flux d'hydrogène pur à 400°C pendant 16 heures. La réaction de synthèse Fischer-Tropsch est opérée dans un réacteur tubulaire de type lit fixe et fonctionnant en continu.

Chacun des catalyseurs se trouve sous forme de poudre de diamètre compris entre 40 et 150 microns.

Les conditions de test sont les suivantes:
- Température = 220°C
- Pression totale = 2MPa
- Vitesse volumique horaire (VVH) = 5000 h⁻¹
- Rapport molaire H₂/CO = 2/1

Les résultats, exprimés en termes d'activité (vitesse de consommation du CO) et de productivité, figurent dans le tableau 3.

**Tableau 3 : performances catalytiques de chaque catalyseur**

| Catalyseur | Vitesse de consommation du CO (10⁻¹g.h⁻¹.g_{cata}⁻¹) après 60h sous flux réactionnel | Productivité massique en C₅⁺ (g.h⁻¹.g_{cata}⁻¹) |
|---|---|---|
| A1 (comparatif) | 5,2 | 0,46 |
| A2 (comparatif) | 4,8 | 0,41 |
| B1 (comparatif) | 6,1 | 0,55 |
| B2 (comparatif) | 6,6 | 0,57 |
| C (invention) | 14,7 | 1,26 |
| D (invention) | 19,5 | 1,46 |
| E (invention) | 11,0 | 0,91 |
| F (invention) | 7,2 | 0,65 |
| G (invention) | 25,8 | 2,27 |
| H (invention) | 5,9 | 0,53 |
| I (invention) | 18,9 | 1,42 |

En comparant des catalyseurs ayant des supports identiques (H envers A1 et A2 ; I envers B1 ; C, D, E, F et G envers B2), les résultats figurant dans le tableau 3 démontrent que les catalyseurs préparés en présence de cyclodextrine sont beaucoup plus actifs que les catalyseurs préparés en l'absence d'un oligosaccharide cyclique. De plus, les catalyseurs préparés en présence de cyclodextrine conduisent à une productivité vers les produits désirés en C5+ supérieure à celle obtenue au moyen des catalyseurs préparés en l'absence d'un oligosaccharide cyclique. Les catalyseurs préparés en présence de cyclodextrine sont donc plus sélectifs envers les produits désirés. L'amélioration des performances catalytiques en termes d'activité et de productivité est sensible en comparant le catalyseur A2 (préparé au moyen de saccharose) avec le catalyseur H (préparé au moyen de bêta-cyclodextrine) : la préparation d'un catalyseur au moyen de la bêta-cyclodextrine, qui est un oligosaccharide cyclique, conduit à l'obtention d'un catalyseur plus actif et plus sélectif envers les produits désirés en C5+ qu'un catalyseur préparé au moyen d'un composé organique qui n'appartient pas à la famille des oligosaccharides cycliques.

## Revendications

1. Procédé de synthèse d'hydrocarbures essentiellement linéaires et saturés C5+ consistant en la mise en contact d'une charge comprenant du gaz de synthèse avec au moins un catalyseur dont la phase active comprend au moins un métal du groupe VIII déposé sur un support formé d'au moins un oxyde, ledit catalyseur étant préparé selon un procédé comprenant au moins :
i) au moins une étape de mise en contact d'au moins dudit support avec au moins une solution contenant au moins un précurseur dudit métal du groupe VIII,
ii) au moins une étape de mise en contact d'au moins dudit support avec au moins un composé organique formé d'au moins un oligosaccharide cyclique composé d'au moins 6 sous-unités glucopyranose liées en α-(1,4),
iii) au moins une étape de calcination pour obtenir au moins ledit métal dudit groupe VIII sous forme oxyde,
les étapes i) et ii) pouvant être réalisées séparément, dans un ordre indifférent, ou simultanément.

2. Procédé de synthèse d'hydrocarbures selon la revendication 1 tel que ladite phase active comprend du cobalt.

3. Procédé de synthèse d'hydrocarbures selon la revendication 1 ou la revendication 2 tel que ladite phase active comprend au moins un métal additionnel choisi parmi le platine, le palladium, le rhénium, le ruthénium, le manganèse et le tantale.

4. Procédé de synthèse d'hydrocarbures selon l'une des revendications 1 à 3 tel que ledit catalyseur présente des cristallites d'oxyde dudit métal du groupe VIII ayant un diamètre moyen d'au moins 6 nm.

5. Procédé de synthèse d'hydrocarbures selon l'une des revendications 1 à 4 tel que ledit support est formé d'au moins un oxyde simple choisi parmi l'alumine (Al₂O₃), la silice (SiO₂), l'oxyde de titane (TiO₂), la cérine (CeO₂) et la zircone (ZrO₂).

6. Procédé de synthèse d'hydrocarbures selon l'une des revendications 1 à 4 tel que ledit support est formé d'une spinelle incluse dans une alumine ou une silice-alumine.

7. Procédé de synthèse d'hydrocarbures selon l'une des revendications 1 à 6 tel que ledit composé organique est choisi parmi les cyclodextrines, les cyclodextrines substituées, les cyclodextrines polymérisées et les mélanges de cyclodextrines.

8. Procédé de synthèse d'hydrocarbures selon la revendication 7 tel que les cyclodextrines sont l'α-cyclodextrine, la β-cyclodextrine et la γ-cyclodextrine respectivement composée de 6, 7 et 8 sous-unités glucopyranose liées en α-(1,4).

9. Procédé de synthèse d'hydrocarbures selon la revendication 7 tel que les cyclodextrines substituées sont l'hydroxypropyl bêta-cyclodextrine et les bêta-cyclodextrines méthylées.

10. Procédé de synthèse d'hydrocarbures selon l'une des revendications 1 à 9 tel que l'introduction dudit composé organique pour la mise en oeuvre de ladite étape ii) est telle que le rapport molaire {(métal(ux) du groupe VIII sous forme d'oxyde présent dans phase active du catalyseur obtenu à l'issue de ladite étape iii)/composé organique} est compris entre 10 et 300.

11. Procédé de synthèse d'hydrocarbures selon l'une des revendications 1 à 10 tel que lorsque lesdites étapes i) et ii) sont réalisées simultanément, la préparation du catalyseur comprend la mise en oeuvre d'une ou plusieurs étapes i).

12. Procédé de synthèse d'hydrocarbures selon l'une des revendications 1 à 10 tel que ladite étape i) est effectuée préalablement à ladite étape ii).

13. Procédé de synthèse d'hydrocarbures selon l'une des revendications 1 à 10 tel que ladite étape ii) est effectuée préalablement à ladite étape i).

14. Procédé de synthèse d'hydrocarbures selon l'une des revendications 1 à 13 tel que ladite étape iii) de calcination est réalisée à une température comprise entre 200 et 800°C

15. Procédé de synthèse d'hydrocarbures selon l'une des revendications 1 à 14 tel qu'il est opéré sous une pression totale comprise entre 0,1 et 15 MPa, sous une température comprise entre 150 et 350°C, la vitesse volumique horaire étant comprise entre 100 et 20000 volumes de gaz de synthèse par volume de catalyseur et par heure (100 à 20000 h⁻¹).

## Patentansprüche

1. Verfahren zur Synthese von überwiegend linearen und gesättigten Kohlenwasserstoffen C5+ bestehend aus dem in Kontaktbringen einer Charge umfassend Synthesegas mit wenigstens einem Katalysator, dessen aktive Phase wenigstens ein Metall der Gruppe VIII umfasst, abgeschieden auf einem durch wenigstens ein Oxid gebildeten Träger, wobei der Katalysator hergestellt ist durch ein Verfahren umfassend wenigstens:
i) wenigstens eine Stufe des in Kontaktbringens wenigstens des Trägers mit wenigstens einer Lösung enthaltend wenigstens eine Vorläuferverbindung des Metalls der Gruppe VIII,
ii) wenigstens eine Stufe des in Kontaktbringens wenigstens des Trägers mit wenigstens einer organischen Verbindung gebildet aus wenigstens einem zyklischen Oligosaccharid, zusammengesetzt aus wenigstens sechs Untereinheiten von Glucopyranose gebunden in α-(1,4),
iii) wenigstens eine Stufe der Kalzination, um wenigstens das Metall der Gruppe VIII in seiner oxidischen Form zu erhalten, wobei die Stufen i) und ii) voneinander getrennt in einer beliebigen Reihenfolge verwirklicht werden können oder simultan.

2. Verfahren zur Synthese von Kohlenwasserstoffen gemäß Anspruch 1, derart, dass die aktive Phase Kobalt enthält.

3. Verfahren zur Synthese von Kohlenwasserstoffen gemäß Anspruch 1 oder Anspruch 2, wobei die aktive Phase wenigstens ein zusätzliches Metall enthält, ausgewählt aus Platin, Palladium, Rhenium, Ruthenium, Mangan und Tantal.

4. Verfahren zur Synthese von Kohlenwasserstoffen gemäß einem der Ansprüche 1 bis 3, bei dem die im Katalysator vorhandenen Oxidkristalle des Metalls der Gruppe VIII einen Durchmesser von wenigstens 6 nm aufweisen.

5. Verfahren zur Synthese von Kohlenwasserstoffen gemäß einem der Ansprüche 1 bis 4, bei dem der Träger gebildet ist aus wenigstens einem einfachen Oxid ausgesucht aus Aluminiumoxid (Al₂O₃), dem Siliciumoxid (SiO₂), dem Titanoxid (TiO₂), dem Ceroxid (CeO₂) und dem Zirkonoxid (ZrO₂).

6. Verfahren zur Synthese von Kohlenwasserstoffen gemäß einem der Ansprüche 1 bis 4, bei dem der Träger gebildet ist durch ein Spinell, inkludiert in einer Tonerde oder einer Silica-Tonerde.

7. Verfahren zur Synthese von Kohlenwasserstoffen gemäß einem der Ansprüche 1 bis 6, bei dem die organische Verbindung gewählt ist aus dem Cyclodextrinen, den substituierten Cyclodextrinen, den polymerisierten Cyclodextrinen und den Mischungen von Cyclodextrinen.

8. Verfahren zur Synthese von Kohlenwasserstoff gemäß Anspruch 7, bei denen die Cyclodextrinen α-Cyclodextrin, β-Cyclodextrin und γ-Cyclodextrin sind, respektive Verbindungen mit 6, 7 und 8 Untereinheiten von Glucopyranose gebunden an α-(1,4).

9. Verfahren zur Synthese von Grundwasserstoffen gemäß Anspruch 7, bei dem die substituierten Cyclodextrine Hydroxipropyl- beta-Cyclodextrin und das methylierte Beta-Cyclodextrin sind.

10. Verfahren zur Synthese von Kohlenwasserstoffen gemäß einem der Ansprüche 1 bis 9, bei dem die organische Verbindungen zur Durchführung der Stufe ii) derart eingeführt wird, dass das Molverhältnis {(Metall(e) der Gruppe VIII in der Form der Oxides anwesend in der aktiven Phase des Katalysators erhalten am Ausgang der Stufe iii))/organische Verbindungen} enthalten ist zwischen 10 und 300.

11. Verfahren zur Synthese von Kohlenwasserstoffen gemäß einem der Ansprüche 1 bis 10, bei dem die Stufen i) und ii) simultan durchgeführt werden, die Herstellung des Katalysators umfasst eine oder mehrere Stufen i).

12. Verfahren zur Synthese von Kohlenwasserstoffen gemäß einem der Ansprüche 1 bis 10, bei denen die Stufe i) vor der Stufe ii) durchgeführt wird.

13. Verfahren zur Herstellung von Kohlenwasserstoffen gemäß einem der Ansprüche 1 bis 10, bei dem die Stufe ii) vor der Stufe i) durchgeführt wird.

14. Verfahren zur Synthese von Kohlenwasserstoffen gemäß einem der Ansprüche 1 bis 13, bei dem die Stufe iii) der Kalzination bei einer Temperatur zwischen 200 und 800 °C durchgeführt wird.

15. Verfahren zur Synthese von Kohlenwasserstoffen gemäß einem der Ansprüche 1 bis 14, durchgeführt bei einem absoluten Druck zwischen 0,1 und 15 MPa, bei einer Temperatur zwischen 150 und 300 °C, einer stündlichen Volumengeschwindigkeit zwischen 100 und 20.000 Volumina Synthesegas pro Volumen Katalysator und pro Stunde (100 bis 20.000 h⁻¹).

## Claims

1. A process for synthesizing essentially linear, saturated C5+ hydrocarbons, consisting of bringing a feed comprising synthesis gas into contact with at least one catalyst the active phase of which comprises at least one metal from group VIII deposited on a support formed by at least one oxide, said catalyst being prepared using a process comprising at least:
i) at least one step for bringing at least said support into contact with at least one solution containing at least one precursor of said metal from group VIII;
ii) at least one step for bringing at least said support into contact with at least one organic compound formed from at least one cyclic oligosaccharide composed of at least 6 α-(1,4)-bonded glucopyranose subunits;
iii) at least one calcining step to obtain at least said metal from group VIII in the oxide form;
the steps i) and ii) possibly being carried out separately, in any order, or simultaneously.

2. A hydrocarbon synthesis process according to claim 1, in which said active phase comprises cobalt.

3. A hydrocarbon synthesis process according to claim 1 or claim 2, in which said active phase comprises at least one additional metal selected from platinum, palladium, rhenium, ruthenium, manganese and tantalum.

4. A hydrocarbon synthesis process according to one of claims 1 to 3, in which said catalyst contains crystallites of oxide of said metal from group VIII with a mean diameter of at least 6 nm.

5. A hydrocarbon synthesis process according to one of claims 1 to 4, in which said support is formed by at least one simple oxide selected from alumina (Al₂O₃), silica (SiO₂), titanium oxide (TiO₂), cerine (CeO₂) and zirconia (ZrO₂).

6. A hydrocarbon synthesis process according to one of claims 1 to 4, in which said support is formed from a spinel included in an alumina or a silica-alumina.

7. A hydrocarbon synthesis process according to one of claims 1 to 6, in which said organic compound is selected from cyclodextrins, substituted cyclodextrins, polymerized cyclodextrins and a mixture of cyclodextrins.

8. A hydrocarbon synthesis process according to claim 7, in which the cyclodextrins are α-cyclodextrin, β-cyclodextrin and γ-cyclodextrin respectively composed of 6, 7 and 8 α-(1,4)-bonded glucopyranose subunits.

9. A hydrocarbon synthesis process according to claim 7, in which the substituted cyclodextrins are hydroxypropyl beta-cyclodextrin and methylated beta-cyclodextrins.

10. A hydrocarbon synthesis process according to one of claims 1 to 9, in which said organic compound for carrying out said step ii) is introduced such that the molar ratio {(metal(s) from group VIII in the oxide form present in the active phase of the catalyst obtained from said step iii)/organic compound} is in the range 10 to 300.

11. A hydrocarbon synthesis process according to one of claims 1 to 10, in which when said steps i) and ii) are carried out simultaneously, the catalyst preparation comprises carrying out one or more steps i).

12. A hydrocarbon synthesis process according to one of claims 1 to 10, in which said step i) is carried out prior to said step ii).

13. A hydrocarbon synthesis process according to one of claims 1 to 10, in which said step ii) is carried out prior to said step i).

14. A hydrocarbon synthesis process according to one of claims 1 to 13, in which said calcining step iii) is carried out at a temperature in the range 200°C to 800°C.

15. A hydrocarbon synthesis process according to one of claims 1 to 14, which is operated at a total pressure in the range 0.1 to 15 MPa, at a temperature in the range 150°C to 350°C, the hourly space velocity being in the range 100 to 20000 volumes of synthesis gas per volume of catalyst per hour (100 to 20000 h⁻¹).
